Europäisches Patentamt

European Patent Office

Office européen des brevets

(19)

(11) Publication number: **0 044 363**
**B1**

# (12) EUROPEAN PATENT SPECIFICATION

(45) Date of publication of patent specification: **23.05.84**

(21) Application number: **80302480.1**

(22) Date of filing: **22.07.80**

(51) Int. Cl.³: **C 07 C 101/18,**
C 07 C 101/24, C 07 C 101/62

(54) **Method for preparing aminoalkyl esters of aminoacids.**

(43) Date of publication of application:
**27.01.82 Bulletin 82/04**

(45) Publication of the grant of the patent:
**23.05.84 Bulletin 84/21**

(84) Designated Contracting States:
**DE FR GB**

(56) References cited:
**DE-A-1 593 963**
**JP-A-46 035 246**

(73) Proprietor: **TORAY INDUSTRIES, INC.**
**2, Nihonbashi Muromachi 2-chome Chuo-ku**
**Tokyo 103 (JP)**

(72) Inventor: **Minami; Muneyoshi**
**24-5, 1-chome Oohira**
**Ootsu-shi, Shiga-ken (JP)**
Inventor: **Saito; Masami**
**836, 1-chome Kokubu**
**Ootsu-shi,Shiga-ken (JP)**

(74) Representative: **Ritter, Stephen David et al**
**Mathys & Squire 10 Fleet Street**
**London EC4Y 1AY (GB)**

Courier Press, Leamington Spa, England.

## Description

This invention relates to a method for preparing aminoalkyl esters of aminoacids.

Aminoalkyl esters of aminoacids on phosgenation afford isocyanates useful as a starting material for polyurethanes.

Aminoalkyl esters of aminoacids can be prepared for example by the method proposed in Japanese Patent Publication No. 35246/1971 wherein, in the esterification reaction of an aminoacid hydrochloride and an aminoalcohol hydrochloride, the esterification is allowed to proceed under heating while passing a stream of hydrogen chloride gas through the reaction system and while removing water as the reaction proceeds. This proposed esterification reaction is carried out in an inert, liquid reaction medium, and the said publication refers to various hydrocarbons and chlorinated hydrocarbons as liquid reaction media. As the esterification proceeds under heating in a liquid reaction medium, water is produced, which together with the reaction medium forms azeotropic system and is distilled out of the reaction system. The water about to be distilled off accompanies hydrogen chloride present in the reaction system as an azeotropic mixture and hydrogen chloride is consumed. To have this esterification reaction proceed, therefore, it has been considered necessary that such a decrease in the amount of hydrogen chloride be compensated for and that the concentration of hydrogen chloride be maintained by passing a stream of hydrogen chloride gas through the reaction system during the reaction.

Having studied the above method, we found that the method in question involves the following demerits: firstly, although the esterification reaction proceeds comparatively favorably in the initial stage of the reaction, the reaction rate gradually decreases, and the conversion reaches near a ceiling at fairly lower yield; secondly, the requirement that the reaction should be carried out while passing a large amount of hydrogen chloride gas causes an operational problem such that the handling of the gas is troublesome.

It is an object of this invention to overcome the above-mentioned demerits and particularly to provide a method whereby the esterification reaction of an aminoacid and an aminoalcohol can be carried out at a higher reaction rate and in a more simplified manner.

According to the present invention there is provided a method for preparing an aminoalkyl ester of amino acid by esterfication of an amino acid hydrochloride and an amino alcohol hydrochloride characterized in that the esterification is effected at a temperature in the range of 50° to 180°C in the absence of an organic solvent capable of forming an azeotropic mixture with water and in the presence of hydrochloric acid absorbed in the reaction mixture, and water produced is intermittently distilled off under reduced pressure.

As indicated above, in the prior art process distilling the produced water out of the reaction system thoroughly was generally considered advantageous for carrying out the reaction favorably. Usually there was adopted an esterification method in which water was distilled off as an azeotropic system in the presence of an organic solvent, then the water thus distilled off is separated and the solvent refluxed.

In contrast therewith, the method of this invention in which the removal of produced water is performed by distilling off under an appropriate reduced pressure, does not allow an organic solvent capable of forming an azeotropic mixture with water to be present in the reaction system, whereby the formation of an azeotropic system is avoided so the reaction mixture is not thoroughly dehydrated but assumes a more incompletely dehydrated state, and then in the latter state the reaction rate becomes surprisingly higher than would be expected.

In this invention, hydrogen chloride gas has to be absorbed by the reaction system, but it is not neccessary for the hydrogen chloride gas to be introduced into the reaction system constantly as a stream of the gas. Therefore, the method of this invention can be carried out even in a closed reaction vessel provided that the surface of reaction mixture is contacted with hydrogen chloride gas.

The produced water is removed outside the reaction system intermittently as necessary.

In the prior art esterification reaction it was considered desirable, as a generally acknowledged opinion, that the produced water be removed from the reaction system as soon as possible. In more particular terms, it was regarded as preferable for the ester-forming compounds to be heated in the presence of an azeotropic organic solvent and for the produced water to be distilled out of the reaction mixture constantly by means of a Dean-Stark water separator trap or the like attached to the reaction vessel. In the method of this invention, unlike such prior proposals, that is, unlike the aforementioned water removal in an azeotropic system, the produced water is not thoroughly removed but is intermittently distilled off under an appropriate reduced pressure and hydrogen chloride gas is brought into direct contact with the reaction mixture after such removal of water thereby having hydrogen chloride more favorably absorbed in the mixture, and thus the reaction is allowed to more fastly proceed.

Accordingly, the remaining water in the reaction mixture is usually larger in amount than in an azeotropic dehydration system, and hydrogen chloride is absorbed more favorably into the reaction mixture, and further its concentration becomes higher. In the esterification reaction system of this invention, usually the ratio of residual water to aminoacid is about several mol% to about 20 mol%, but in the initial stage of the reaction it may be about 40 mol%. The concentration of hydrogen chloride in the reaction mixture usually is a few mol% to about 8 mol%, but a higher concentration even to the vicinity of 15 mol% is also reached. On the other hand, in the conventional azeotropic system using an organic solvent, the concentration of hydrogen chloride in reaction mixture is at most near 1 mol% or so in many cases.

The reaction accelerating effect in the esterification reaction of this invention is presumed to be based firstly on the effect of hydrogen chloride as a catalyst but also on the catalytic effect of water itself, although this effect has not yet been so certainly clarified. In any case, as compared with the conventional esterification methods such as the one previously referred to in which the reaction is carried out in an azeotropic dehydration system in the presence of an organic solvent capable of forming an azeotropic mixture with water while having hydrogen chloride absorbed in the reaction system, the esterifying method of this invention has exhibited unexpected effects in the preparation of aminoalkyl esters of aminoacids in which the esterification is allowed to proceed in the absence of an organic solvent capable of forming an azeotropic mixture with water and in the presence of hydrogen chloride gas absorbed directly in the reaction system while the produced water is removed intermittently to an incomplete extent by distillation under reduced pressure.

Aminoacid hydrochlorides which may be used in this invention are hydrochlorides of aliphatic or aromatic aminoacids having at least one amino group and at least one carboxylic group and having 2 to 18 carbon atoms, or of lactams of 3 to 12-membered ring. Particularly preferred aminoacids are aliphatic monoaminomonocarboxylic acids, diamino-monocarboxylic acids, monoaminodicarboxylic acids, and diamino-dicarboxylic acids. Lactams resulting from cyclization of these aminoacids are also preferably used. To exemplify the above compounds, mention may be made to glycine, 3-aminopropionic acid, ω-aminocaproic acid, ω-aminolauric acid, alanine, isoleucine, 3-aminobutyric acid, 4-aminocyclohexane-carboxylic acid, phenylalanine, methionine, aminobenzoic acid, aspartic acid, glutamic acid, lysine, lanthionine, 1-amino-2,3,4-butane-tricarboxylic acid, as well as lactams of these aminoacids such as pyrrolidone, caprolactam and laurolactam. It goes without saying that these compounds may contain functional groups inert to the esterification reaction, for example, such substituent groups as alkyl, cycloalkyl, aryl, halogen, and so on.

Aminoalcohol hydrochlorides which may be used in the invention are hydrochloride of aminoalcohols having one primary or secondary hydroxyl group and one primary amino group and having 2 to 12 carbon atoms. These aminoalcohols may contain, in the alkylene chain thereof, hetero atoms such as oxygen and sulfur, or groups inert to the esterification reaction, for example, such substituent groups as alkyl, cycloalkyl, aryl, nitro, halogen, and so on. By way of illustrating such aminoalcohols, mention may be made of ethanolamine, 1-amino-2-propanol, 2-amino-1-propanol, 2-aminoisobutanol, 2-amino-1-butanol, 2-(2-aminoethoxy)-ethanol, and 2-aminocyclo-hexanol.

In the esterification reaction, the molar ratio of an aminoacid hydrochloride to an aminoalcohol hydrochloride is preferably in the range of 1:0.8 to 1:8, more preferably 1:1.2 to 1:3, and the heating temperature is in the range of 50° to 180°C, preferably 80° to 150°C. If an aminoalcohol hydrochloride is used in a larger amount than the above range, the esterification reaction will be accelerated, but at the same time there will occur an undesired side reaction which produces water, resulting in that it becomes difficult to isolate the object substance. Therefore, it is suitable that an aminoalcohol hydrochloride be used in an amount within the above range of molar ratio. The reaction may be carried out under normal pressure and also under pressure or under somewhat reduced pressure.

For the purification of product there may be used various known methods, but with respect to the ester from lysine dihydrochloride and ethanolamine hydrochloride, the purification is effectively carried out by using a mixed methanol-ethanol solvent as a recrystallization solvent.

The products, which usually are obtained in the form of hydrochlorides according to the method of the invention, can be converted to the free compounds by any of the processes known in the art, such as neutralization of the strong acid, extraction, heating to the salt dissociation temperature, and the like. Therefore, the "aminoalkyl esters of aminoacids" as referred to herein include salts thereof.

Working examples of this invention are given below to further illustrate the invention.

Example 1
210 g. (1 mole) of a synthesized 2-aminocaprolactam (61% aqueous solution) was mixed with 406 g. (4 moles) of a concentrated hydrochloric acid (36%), and the resulting solution was heated at 97°C for 10 hours to allow hydrolysis to take place. Then, 122 g. (2 moles) of ethanolamine was added, and a reaction mixture solution of lysine · 2HCl (1 mole) and ethanolamine · HCl (2 moles) is prepared and at a bath temperature of 80°C the solution was concentrated under reduced pressure first by a water-jet pump and lastly by a vacuum pump to a pressure of 2—3 mmHg; as a result, there was obtained a reaction mixture in a molten state with a water content below 0.5 wt%. The reaction mixture was placed in a closed, 1 litre flask equipped with a stirrer, then the inside temperature of the flask was raised to 120°C and the interior was first reduced in pressure to exhaust the air therefrom, then purged with hydrogen chloride gas to allow the gas to come into contact with the reaction mixture. For 10 to 15 minutes hydrogen chloride was allowed to be absorbed in the reaction mixture with stirring. During this period, replenishment of the hydrogen chloride gas absorbed was ensured by connecting the flask to an exterior, hydrogen chloride gas reservoir. Thereafter, for a very short time, the interior of the flask was reduced in pressure to 300—400 mmHg for exhaustion of gas, then purged with air. This operation is repeated two or three times, then the flask is allowed to stand at 120°C, and after the lapse of 40 to 60 minutes the produced water is distilled off at a reduced pressure below 10 mmHg. After repeating this operation five or six times, the esterification rate reaches 75% to 80%. In the above esterifying operation, as to lysine 1 mole the residual amount of water after dehydration under reduced pressure was about 0.05 to 0.15 mole, and the amount of the hydrogen chloride gas absorbed was 0.05 to 0.08 mole. The absorption of hydrogen chloride gas may be increased to 0.1 to 0.15 mole by making the absorption time longer. From this reaction product was isolated 190 g. of a purified 2-aminoethyl-2,6-diamino-caproate by recrystallization from a mixed methyl alcohol—ethyl alcohol solvent, m.p. 143—144°C.

Example 2

219 g (1 mole) of L-lysine dihydrochloride and 196 g. (2 moles) of ethanolamine hydrochloride were thoroughly pulverized and mixed together, then dried under reduced pressure for 3 hours at 70°C. The mixture was placed in a closed, 1 litre flask, then the inside temperature of the flask was raised to 120°C and, in the same manner as in Example 1, hydrogen chloride gas was allowed to be absorbed in the molten mixture, under which condition reaction was allowed to proceed. After standing for such a short time of about 30 minutes as the reaction time, the esterification rate is surprisingly accelerated and the yield reached so high 58%.

Example 3

Ethanolamine hydrochloride was reacted with each of ω-aminocaproic acid hydrochloride and 3-aminopropionic acid hydrochloride in the same manner as in Example 2 except that the reaction operation was repeated several times. As a result, it was confirmed that esterification took place very rapidly, as was the case with Example 2.

Example 4

In the same manner as in Example 3, lysine dihydrochloride was reacted with each of 2-amino-1-propanol hydrochloride and 1-amino-2-propanol hydrochloride, to find that esterification took place very rapidly as was the case with Example 2.

Reference Example

An aqueous solution of a reaction mixture which had been prepared in the same manner as in Example 1 was placed in a four-necked, 1 litre flask, in which was further placed a mixed toluene-xylene solvent (b.p. 120°C). To the flask was attached a hydrogen chloride gas introducing pipe and were also attached, for distilling off water continuously in an azeotropic system, a reflux condenser and a water separate. While passing in hydrogen chloride gas at a rate of 50 cc/min., heat was applied with stirring. At an inside temperature of about 90°C water was distilled off azeotropically. About 4 hours was required to distill off 324 g. of water which was present from the beginning. The conversion at this time was 16%, thereafter about 40% in 5 hours, 55% in 15 hours, and 70% in 25 hours. Thus the reaction velocity was very low. The amount of hydrogen chloride absorbed in this reaction mixture was 0.008 to 0.012 mole to lysine 1 mole.

**Claims**

1. Method for preparing an aminoalkyl ester of amino acid by esterification of an amino acid hydrochloride and an amino alcohol hydrochloride characterized in that the esterification is effected at a temperature in the range of 50° to 180°C in the absence of an organic solvent capable of forming an azeotropic mixture with water and in the presence of hydrochloric acid absorbed in the reaction mixture, and water produced is intermittently distilled off under reduced pressure.

2. A method according to claim 1, in which the amino acid is selected from aliphatic and aromatic amino acids having at least one amino group and at least one carboxyl group and having 2 to 18 carbon atoms, and lactams of 3 to 12-membered ring.

3. A method according to claim 1, in which the amino alcohol is selected from amino alcohols containing 2 to 12 carbon atoms and having one primary or secondary hydroxyl group and one primary amino group.

4. A method according to claim 1, in which the amino acid is 2-aminocaprolactam.

5. A method according to claim 1, in which the amino acid is lysine.

6. A method according to claim 1, in which the amino acid is ω-aminocaproic acid.

7. A method according to claim 1, in which the amino acid is 3-aminopropionic acid.

8. A method according to claim 1, in which the amino alcohol is ethanolamine.

9. A method according to claim 1, in which the amino alcohol is 1-amino-2-propanol.

10. A method according to any of claims 1 to 9, in which the molar ratio of the amino acid hydrochloride to the amino alcohol hydrochloride is in the range of from 1:0.8 to 1:8.

## Patentansprüche

1. Verfahren zur Herstellung eines Aminosäureaminoalkylesters durch Veresterung eines Aminosäurehydrochlorids mit einem Aminoalkoholhydrochlorid, dadurch gekennzeichnet, daß die Veresterung bei einer Temperatur in dem Bereich von 50 bis 180°C in Abwesenheit eines organischen Lösungsmittels, das mit Wasser eine azeotrope Mischung bilden kann, und in Gegenwart von in der Reaktionsmischung absorbierter Chlorwasserstoffsäure durchgeführt wird, und daß das gebildete Wasser intermittierend unter vermindertem Druck abdestilliert wird.

2. Verfahren nach Anspruch 1, worin die Aminosäure ausgewählt wird aus aliphatischen und aromatischen Aminosäuren mit mindestens einer Aminogruppe und mindestens einer Carboxylgruppe und 2 bis 18 Kohlenstoffatomen und Lactamen mit einem 3- bis 12-gliedrigen Ring.

3. Verfahren nach Anspruch 1, worin der Aminoalkohol ausgewählt wird aus Aminoalkoholen mit 2 bis 12 Kohlenstoffatomen und mit einer primären oder sekundären Hydroxylgruppe und einer primären Aminogruppe.

4. Verfahren nach Anspruch 1, worin die Aminosäure 2-Aminocaprolactam ist.

5. Verfahren nach Anspruch 1, worin die Aminosäure Lysin ist.

6. Verfahren nach Anspruch 1, worin die Aminosäure $\omega$-Aminocapronsäure ist.

7. Verfahren nach Anspruch 1, worin die Aminosäure 3-Aminopropionsäure ist.

8. Verfahren nach Anspruch 1, worin der Aminoalkohol Ethanolamin ist.

9. Verfahren nach Anspruch 1, worin der Aminoalkohol 1-Amino-2-propanol ist.

10. Verfahren nach einem der Ansprüche 1 bis 9, worin das Molverhältnis von Aminosäurehydrochlorid zu Aminoalkoholhydrochlorid in dem Bereich von 1:0,8 bis 1:8 liegt.

## Revendications

1. Procédé pour préparer un ester aminoalkylique d'aminoacide par estérification d'un chlorhydrate d'aminoacide et d'un chlorhydrate d'aminoalcool, caractérisé en ce que l'estérification est effectuée à une température dans l'intervalle de 50 à 180°C, en l'absence d'un solvant organique capable de former un mélange azéotropique avec l'eau et en présence d'acide chlorhydrique absorbé dans le mélange réactionnel, et l'eau produite est retirée par distillation par intermittence sous pression réduite.

2. Procédé selon la revendication 1, dans lequel l'aminoacide est choisi parmi des aminoacides aliphatiques et aromatiques ayant au moins un groupe amino et au moins un groupe carboxyle ayant 2 à 18 atomes de carbone, et des lactames à noyau triangulaire à dodécagonal.

3. Procédé selon la revendication 1, dans lequel l'aminoalcool est choisi parmi des aminoalcools contenant 2 à 12 atomes de carbone et ayant un groupe hydroxyle primaire ou secondaire et un groupe amino primaire.

4. Procédé selon la revendication 1, dans lequel l'aminoacide est le 2-aminocaprolactame.

5. Procédé selon la revendication 1, dans lequel l'aminoacide est la lysine.

6. Procédé selon la revendication 1, dans lequel l'aminoacide est l'acide $\omega$-aminocaproïque.

7. Procédé selon la revendication 1, dans lequel l'aminoacide est l'acide 3-aminopropionique.

8. Procédé selon la revendication 1, dans lequel l'aminoalcool est l'éthanolamine.

9. Procédé selon la revendication 1, dans lequel l'aminoalcool est le l'-amino-2-propanol.

10. Procédé selon l'une quelconque des revendications 1 à 9, dans lequel le rapport molaire chlorhydrate d'aminoacide/chlorhydrate d'aminoalcool est dans la gamme de 1:0,8 à 1:8.